# EUROPÄISCHE PATENTSCHRIFT

(11) **EP 2 572 739 B1**
(45) Veröffentlichungstag und Bekanntmachung des Hinweises auf die Patenterteilung: **22.10.2014**
(21) Anmeldenummer: 12005649.4
(22) Anmeldetag: 03.08.2012
(51) Int. Cl.: C02F 1/44, C02F 1/00, A61M 1/16, B01D 61/12, B01D 65/02, B01D 61/08, B01D 61/02, B01D 65/10, C02F 103/02, C02F 103/04

(54) **Vorrichtung zur Erzeugung von Reinstwasser**
Device for producing ultra-pure water
Dispositif de production d'eau ultrapure

(30) Priorität: 24.09.2011 DE 102011114912
(43) Veröffentlichungstag der Anmeldung: 27.03.2013
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- EP-A1- 2 067 520
- EP-A1- 2 145 637
- EP-A1- 2 423 169
- WO-A1-2011/132016
- WO-A1-2012/053225
- WO-A2-2007/087578
- WO-A2-2008/089913

## Beschreibung

Die Erfindung betrifft eine Vorrichtung zur Wasseraufbereitung nach dem Prinzip der umgekehrten Osmose. Vorrichtungen dieser Art, Umkehrosmose- oder RO-Anlagen, werden insbesondere in Verbindung mit Hämodialysegeräten eingesetzt, um aus Leitungswasser hochreines, keimfreies Wasser zur Bereitung der Dialysierflüssigkeit zu gewinnen.

Das Funktionsprinzip von Umkehrosmoseanlagen besteht bekanntlich darin, dass das aufzubereitende Wasser in einem Filtermodul unter hohem Druck an der Oberfläche einer semipermeablen Membran entlanggeführt wird, wobei ein Teil des Wassers, das sogenannte Permeat, durch die Membran tritt und auf der anderen Seite der Membran gesammelt und den Verbrauchsstellen zugeführt wird. Der nicht durch die Membran tretende, mit zurückgehaltenen Stoffen angereicherte Teil des Rohwassers, das sogenannte Konzentrat, fließt am Ende der Strömungsstrecke des Primärraumes aus dem Membranmodul aus.

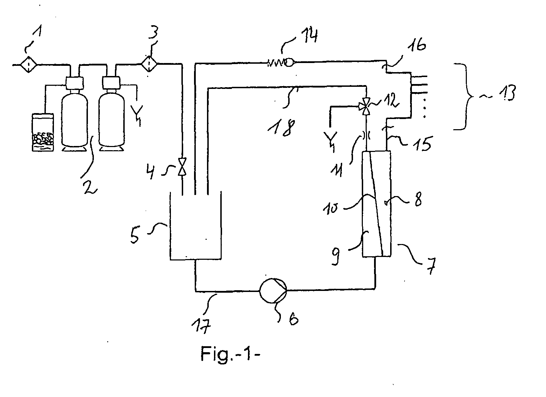

Das in Fig. 1 gezeigte Schema veranschaulicht als typisches Beispiel das Zusammenwirken wesentlicher Funktionselemente einer Umkehrosmoseanlage nach dem Stand der Technik. Das aufzubereitende Rohwasser fließt aus der zuführenden Leitung 1 und über das Ventil 4 in ein Puffergefäß 5 mit eingebauter Füllstandsregelung. Aus diesem Behälter 5 gelangt das Wasser durch die Leitung 17 über die Pumpe 6 in den Umkehrosmosefilter 7, dessen Primärraum 9 durch die semipermeable Membran 10 von dem Sekundärraum 8 getrennt ist. Aus dem Sekundärraum 8 fließt das Permeat in eine Ringleitung 15/16 von der die Verbraucherleitungen 13 abzweigen. Überschüssig erzeugtes Permeat kann am Ende der Ringleitung über ein eingefügtes Druckhalteventil 14 in das Gefäß 5 zurückfließen, wobei die Einstellung dieses Ventils den in der Ringleitung 15/16 herrschenden Druck bestimmt.

Der für die Filtration notwendige Druck im Primärraum des RO-Filters 9 wird durch die Pumpe 6 in Verbindung mit einem in die Konzentratleitung 18 stromabwärts vom Filter eingefügten Strömungswiderstand 11, z.B. in Form eines Drossel- oder eines Druckventils, erzeugt.

Für die Funktion des RO Filters 7 ist die Konzentrationsdifferenz zurückgehaltener Stoffe zwischen Auslass und Einlass des Primäraumes 9 von erheblicher Bedeutung. Bei zu hoher Konzentration insbesondere von Kalzium und Magnesium besteht ein erhöhtes Risiko, dass diese Anteile eine kritische Grenze überschreiten. Durch Bildung von Ablagerung nimmt dann die Durchlässigkeit der Membran 10 und damit der Permeatfluss ab, was einem vorzeitigen Unbrauchbarwerden des Umkehrosomosefilters entspricht.

Aufgrund dieses Sachverhaltes werden bisher unter Berücksichtigung der Rohwasserqualität insbesondere die Kalzium- und Magnesiumsalze durch vorgeschaltete Kationentauschersäulen 2 gegen Natrium ausgetauscht. Ionenaustauscher sind wartungs- u. kostenintensiv.

Zum sicheren Betrieb der Kationentauscher ist Natriumchlorid und Spülwasser erforderlich. Darüber hinaus muss regelmäßig manuell Salz nachgefüllt werden. Zusätzlich kontaminiert das salzhaltige Spülwasser die Abwässer.

Umkehrosomosen dienen insbesondere auch der Gewinnung von keimfreiem Wasser.

Der nicht durch die Membran 10 tretende, mit zurückgehaltenen chem. Wasserinhaltsstoffen und Bakterien angereicherte Teil des zugeführten Leitungswassers bildet einen Biofilm auf den Innenflächen des flüssigkeitsführenden Systems. Die Ausscheidungen des Biofilms können als Pyrogene und Endotoxine die nicht ideale Membran 10 passieren und kontaminieren den hochreinen Permeatkreislauf 15/16.

Daher ist es bisher üblich, an Umkehrosmosen in regelmäßigen Zeitabständen eine thermische oder chemische Desinfektion durchzuführen. Hierzu wird der Betrieb unterbrochen und der Anlage thermische Energie oder chem. Desinfektionsmittel zugeführt.

Wegen der erheblichen Gefahren, die insbesondere mit einer chem. Desinfektion verbunden sind, müssen die Arbeitsschritte hierbei manuell überwacht werden. Das bedeutet im allgemeinen einen erheblichen Arbeitsaufwand.

WO 2007/087578 A2 offenbart ein System zur Überwachung von Umkehrosmosemembranen, bei dem mit Hilfe eines Bildgebungssystems aus Mikroskop und Kamera organische und anorganische Ablagerungen erfasst werden. EP 2 145 637 A1 beschreibt eine Absaugpumpeneinheit, bei der mit Hilfe eines Spektrometers Ablagerungen erfasst werden. EP 2 067 520 A1 offenbart eine Filteranlage mit Umkehrosmosemembranen, die ein Biofilmevaluierungsgerät aufweist, mit dem organische Ablagerungen erfasst werden. EP 2 423 169 A1 betrifft eine Vorrichtung zur Erzeugung von Reinstwasser mit Umkehrosmose und Enthärter, die eine elektrooptische Sender-Empfänger-Einheit zur Erfassung von Ablagerungen aufweist. WO 2012/053225 A1 offenbart eine Membrantrennvorrichtung mit einer Einrichtung zur Erfassung von Licht von einem Submodul, um die Dicke einer Ablagerung zu bestimmen. WO 2011/132016 A1 offenbart ein System zur Optimierung eines Membranreinigungsprozesses mit einem Membraneffizienzmessgerät über die Ultraschallmethode.

Aufgabe der Erfindung ist es, die Betriebskosten dadurch zu reduzieren, dass automatisch ermittelt wird, wann eine chemische und/oder thermische Desinfektion bzw. Reinigung des Primärkreises und/oder Sekundärkreises bzw. deren Leitungen und Komponenten erforderlich ist.

Diese Aufgabe wird erfindungsgemäß durch die Merkmale des Patentanspruchs 1 gelöst.

Vorteilhafte Weiterbildungen der Erfindung sind in den Unteransprüchen gekennzeichnet.

Die Erfindung sieht vor, dass in oder an dem Primärkreis und/oder dem Sekundärkreis eine Einrichtung zur Erfassung von organischen und/oder anorganischen Ablagerungen angeordnet ist, die mit einer Auswerteeinrichtung verbunden ist. Die Einrichtung erfasst Ablagerungen in der zugehörigen Flüssigkeitsleitung, vorzugsweise an deren Innenwand, oder an einer Komponente des Primärkreises oder Sekundärkreises, beispielsweise innerhalb des Filters, und gibt die erfassten Messwerte an eine Auswerteeinrichtung weiter, die in die Steuerungseinrichtung der RO-Anlage oder eines angeschlossenen Dialysegeräts integriert sein kann. Die Auswerteeinrichtung ist zweckmäßigerweise mit einer Anzeigeeinrichtung für die ermittelten Messwerte der eine Verunreinigung anzeigenden Ablagerungsschicht versehen.

Außerdem ist erfindungsgemäß vorgesehen, dass in den Sekundärkreis ein Puffergerät aus einem flexiblen, expandierbaren Material eingebaut ist, das zur Rückspülung der Membran mit Permeat geeignet ist, wobei die Einrichtung zur Erfassung von organischen und/oder anorganischen Ablagerungen geeignet ist, bei entsprechender Verunreinigung die Rückspülung einzuleiten.

Weiter ist mit Vorteil vorgesehen, dass für die ermittelten Messwerte ein Grenzwert vorgegeben ist, bei dessen Erreichen (oder Überschreiten) ein Warnsignal erzeugt wird, das anzeigt, dass eine chemische und/oder thermische Desinfektion der Vorrichtung durchgeführt werden muss.

Auf diese Weise werden diese Reinigungsvorgänge nicht - wie dies bisher üblich ist - zu starr vorgegebenen Zeitpunkten durchgeführt, sondern dann wenn die ermittelten Ablagerungen anzeigen, dass ein solcher Reinigungsvorgang nun notwendig ist. Damit lassen sich einerseits zu häufige Reinigungsvorgänge vermeiden, und es ist andererseits sicher gestellt, dass bei einer unvorhergesehen starken Verunreinigung, für die mannigfaltige Gründe in Betracht kommen, der notwendige Reinigungsvorgang durchgeführt wird.

Mit großem Vorteil ist vorgesehen, dass die Einrichtung zur Erfassung von Ablagerungen eine Sensoreinrichtung aufweist, die einen Sender zur Aussendung von optischen Signalen und einen Empfänger für die Signale aufweist.

Dabei kann auch vorgesehen sein, dass die von dem Sender ausgesandten optischen Signale von einer gegenüber liegenden Spiegeleinrichtung zu dem optischen Empfänger zurück gestrahlt werden.

Die Quantität des Empfängersignals ist eine direkte Funktion des Verschmutzungsgrades oder der Dicke der Ablagerungsschicht.

Um die Ablagerung von biologischen Schmutzschichten zu bestimmen, kann auch vorgesehen sein, dass diese Ablagerung durch Anstrahlung beispielsweise mit UV-Licht ein der Schichtdicke entsprechendes fluoreszierendes messbares Antwortsignal reflektiert.

Die flüssigkeitsführende Leitung, die mit der Sensoreinrichtung versehen ist, kann mit transparentem oder transluzentem Material ausgestaltet sein, wodurch der Sender, der Empfänger und gegebenenfalls die Spiegeleinrichtung außerhalb der Leitung einander gegenüberliegend angeordnet sein können.

Die Verunreinigung insbesondere durch eine Kalkablagerung kann auch auf andere Weise als durch eine Sensoreinrichtung ermittelt werden. Bei heiß reinigbaren Anlagen mit bekanntem Volumen kann die Verschmutzung, insbesondere die Kalkablagerung, auf der Heizungsoberfläche in der Weise ermittelt werden, dass der Energieeintrag als Maß der Verschmutzung ausgewertet wird, da bei einer verschmutzten, bzw. mit Ablagerung versehenen Heizoberfläche der Wärme- bzw. Energieeintrag in die Flüssigkeit länger dauert.

Mit Vorteil wird die Sensorik in eine Messkammer eingebaut, in der sich neben einer Messoberfläche eine gegen Verunreinigung inerte Oberfläche befindet, die als Kalibrier und Vergleichsoberfläche die relative Verschmutzung festlegt.

Die Erfindung ermöglicht es, durch Vorgabe von durch Versuche zu ermittelnden Grenzwerten von Ablagerungen an Permeat oder Konzentrat führenden Leitungen bzw. Komponenten die Zeitpunkte zu ermitteln, an denen ein chemischer oder physikalischer Reinigungsvorgang des Leitungssystems des Umkehrosmosegerätes erforderlich ist. Dies erhöht die Sicherheit der Vorrichtung und vermeidet unnötige Betriebskosten.

Dabei ist eine besonders effektive Form der Membranreinigung die Umkehrung der Filtrationsrichtung an der semipermeablen Membrane 10, mittels eines negativen Transmembrandruckes. zur Erzeugung eines negativen Transemembrandruckes wird ein Puffergefäß mit flexiblen, expandierbaren Volumen in die Permeatleitung 15 eingebaut.

Bei entsprechender Verunreinigung gibt die Sensorik die Rückspülung frei, indem die Pumpe stoppt, Ventil 12 zum Abfluss hin öffnet. Vorzugsweise wird dabei der Flusswiderstand 11 geöffnet oder mittels eines Bypassventils überbrückt. Das vorgespannte Volumen im Puffergefäß fließt dabei über die Membran 10 zum Abfluss und versorgt gleichzeitig die Verbraucher 13.

Nach kurzer Rückspülung wird durch Einschalten der Pumpe 6 der Normalbetrieb wieder aufgenommen.

Es wird betont, dass die Erfindung nicht auf die beschriebenen und dargestellten Ausführungsformen beschränkt ist. Vielmehr sind alle offenbarten Merkmale der Ausführungsformen auch einzeln untereinander anders als oben beschrieben miteinander kombinierbar. Die nachfolgenden Patentansprüche definieren die Erfindung.

## Patentansprüche

1. Vorrichtung zur Erzeugung von Reinstwasser nach dem Prinzip einer umgekehrten Osmose mit einem Umkehrosmosefilter (7), der durch die RO-Membran (10) in einen Primärraum (9) und einen Sekundärraum (8) unterteilt ist, mit einem Primärkreis (17, 18), durch den Rohwasser dem Primärraum (9) zugeführt und Konzentrat daraus abgeführt wird, sowie einem Sekundärkreis (15, 16) zur Zufuhr von Permeat zu wenigstens einem Verbraucher, vorzugsweise einem Dialysegerät, wobei in die Leitung (17) des Primärkreises (17,18) eine Pumpe (6) und in die Konzentratleitung (18) des Primärkreises (17, 18) ein Strömungswiderstand (11) und ein Ventil (12) mit einem Abfluss eingeschaltet sind, und wobei in oder an dem Primärkreis (17,18) und/oder dem Sekundärkreis (15,16) eine Einrichtung zur Erfassung von organischen und/oder anorganischen Ablagerungen angeordnet ist, die mit einer Auswerteeinrichtung verbunden ist,
**dadurch gekennzeichnet,**
**dass** in die Permeatleitung (15) des Sekundärkreises (15,16) ein Puffergefäß aus einem flexiblen, expandierbaren Material zur Erzeugung eines negativen Transmembrandrucks eingebaut ist, wobei eine Sensorik der Einrichtung zur Erfassung von organischen und/oder anorganischen Ablagerungen bei entsprechender Verunreinigung die Rückspülung der Membran freigibt, indem die Pumpe (6) stoppt und das Ventil (12) zum Abfluss geöffnet wird.

2. Vorrichtung nach Anspruch 1,
**dadurch gekennzeichnet,**
**dass** die Auswerteeinrichtung mit einer Anzeigeeinrichtung für die Messwerte verbunden ist.

3. Vorrichtung nach Anspruch 1 oder 2,
**dadurch gekennzeichnet,**
**dass** die Auswerteeinrichtung bei Erreichen oder Überschreiten eines vorgegebenen Grenz-Messwertes ein Warnsignal abgibt.

4. Vorrichtung nach Anspruch 1,
**dadurch gekennzeichnet,**
**dass** die Einrichtung zur Erfassung von Ablagerungen eine Sensorik mit einem Sender zur Aussendung von optischen Signalen und einem Empfänger für ankommende Signale aufweist.

5. Vorrichtung nach Anspruch 4,
**dadurch gekennzeichnet,**
**dass** dem Sender der optischen Signale eine die Signale reflektierende Spiegeleinrichtung als Messoberfläche gegenüber liegt, die die Signale zu dem Empfänger reflektiert.

6. Vorrichtung nach Anspruch 5,
**dadurch gekennzeichnet,**
**dass** sich neben der Messoberfläche eine gegen Ablagerung inerte Oberfläche als Vergleichs- oder Kalibrieroberfläche befindet.

7. Vorrichtung nach einem der Ansprüche 4 bis 6,
**dadurch gekennzeichnet,**
**dass** der die Sensorik enthaltende Abschnitt der die Flüssigkeit führenden Leitung oder der mit der Flüssigkeit in Berührung kommenden Komponente ganz oder teilweise aus transparentem oder transluzentem Material besteht.

8. Vorrichtung nach einem der Ansprüche 4 bis 7,
**dadurch gekennzeichnet,**
**dass** die Sensorik in eine Messkammer eingebaut ist.

9. Vorrichtung nach einem der Ansprüche 1 bis 8,
**dadurch gekennzeichnet,**
**dass** zur Erfassung von biologischen Ablagerungen UV-Licht ausgesandt wird.

10. Vorrichtung nach einem der Ansprüche 1 bis 9,
**dadurch gekennzeichnet,**
**dass** die Auswerteeinrichtung die Dicke der biologischen und/oder anorganischen Ablagerung bestimmt.

## Claims

1. Apparatus for producing ultrapure water on the reverse osmosis principle with a reverse osmosis filter (7), which is divided by the RO membrane (10) into a primary space (9) and a secondary space (8), a primary circuit (17, 18), through which the raw water is supplied to the primary space (9) and concentrate is discharged from it, and a secondary circuit (15, 16) for supplying permeate to at least one consumer, preferably a dialysis device, wherein connected into the conduit (17) of the primary circuit (17, 18) there is a pump (6) and into the concentrate conduit (18) of the primary circuit (17, 18) there is a flow resistance (11) and a valve (12) with a discharge, and wherein arranged in or on the primary circuit (17, 18) and/or the secondary circuit (15, 16) there is a device for detecting organic and/or inorganic deposits, which is connected to an analysis device, **characterised in that** the incorporated into the permeate conduit (15) of the secondary circuit (15, 16) there is a buffer vessel of a flexible, expandable material for producing a negative transmembrane pressure, wherein a sensor system of the device for detecting organic and/or inorganic deposits enables the back flushing of the membrane if appropriate contamination is present by stopping the pump (6) and opening the valve (12) to discharge.

2. Apparatus as claimed in claim 1, **characterised in that** the analysis device is connected to an indicating device for the measured values.

3. Apparatus as claimed in claim 1 or 2, **characterised in that** the analysis device emits a warning signal when a predetermined threshold measurement value is reached or exceeded.

4. Apparatus as claimed in claim 1, **characterised in that** the device for detecting deposits includes a sensor system with a transmitter for emitting optical signals and a receiver for incoming signals.

5. Apparatus as claimed in claim 4, **characterised in that** a mirror device, which reflects the signals and constitutes a measurement surface, which reflects the signals to the receiver, is opposed to the sender of the optical signals.

6. Apparatus as claimed in claim 5, **characterised in that** situated adjacent the measurement surface is a surface which is inert to deposits and constitutes a comparison or calibration surface.

7. Apparatus as claimed in one of claims 4 to 6, **characterised in that** the section of the conduit conducting the liquid including the sensor system or the component coming into contact with the liquid consists wholly or partially of transparent or translucent material.

8. Apparatus as claimed in one of claims 4 to 7, **characterised in that** the sensor system is incorporated into a measuring chamber.

9. Apparatus as claimed in one of claims 1 to 8, **characterised in that** UV light is emitted for the detection of biological deposits.

10. Apparatus as claimed in one of claims 1 to 9, **characterised in that** the analysis device determines the thickness of the biological and/or inorganic deposit.

## Revendications

1. Dispositif de production d'eau extra-pure selon le principe d'une osmose inverse, comprenant un filtre à osmose inverse (7), qui est divisé par la membrane d'osmose inverse (10) en un espace primaire (9) et un en espace secondaire (8), comprenant un circuit primaire (17, 18), lequel permet d'amener de l'eau brute à l'espace primaire (9) et d'évacuer de ce dernier un concentré, comprenant également un circuit secondaire (15, 16) servant à amener un perméat à au moins un consommateur, de préférence à un appareil de dialyse, sachant qu'une pompe (6) est installée dans la conduite (17) du circuit primaire (17, 18) et qu'une résistance à l'écoulement (11) et une soupape (12) dotée d'une sortie pour flux sortant sont installées dans la conduite pour concentré (18) du circuit primaire (17, 18), et sachant qu'un système servant à détecter des dépôts organiques et/ou inorganiques est disposé dans ou au niveau du circuit primaire (17, 18) et/ou dans ou au niveau du circuit secondaire (15, 16), ledit système étant connecté à un système d'analyse,
**caractérisé en ce que**
un récipient tampon fabriqué à partir d'un matériau flexible, expansible et servant à produire une pression transmembranaire négative est placé dans la conduite pour perméat (15) du circuit secondaire (15, 16), sachant que des capteurs du système servant à détecter des dépôts organiques et/ou inorganiques active, en cas de contamination correspondante, le rinçage de la membrane, **en ce que** la pompe (6) est arrêtée et la soupape (12) est ouverte aux fins de l'évacuation du flux sortant.

2. Dispositif selon la revendication 1,
**caractérisé en ce que**
le système d'analyse est connecté à un système d'affichage des valeurs de mesure.

3. Dispositif selon la revendication 1 ou 2,
**caractérisé en ce que**
le système d'analyse émet un signal d'avertissement lorsqu'une valeur de mesure limite prédéfinie est atteinte ou dépassée.

4. Dispositif selon la revendication 1,
**caractérisé en ce que**
le système servant à détecter des dépôts présente des capteurs dotés d'un émetteur pour envoyer des signaux optiques et dotés d'un récepteur pour les signaux entrants.

5. Dispositif selon la revendication 4,
**caractérisé en ce que**
un système à miroir réfléchissant les signaux fait face, en tant que surface de mesure, à l'émetteur des signaux optiques, ledit système à miroir réfléchissant les signaux en direction du récepteur.

6. Dispositif selon la revendication 5,
**caractérisé en ce que**
une surface inerte par rapport au dépôt se trouve, en tant que surface de comparaison ou surface d'étalonnage, à côté de la surface de mesure.

7. Dispositif selon l'une quelconque des revendications 4 à 6,
**caractérisé en ce que**
la section, comprenant les capteurs, de la conduite acheminant le liquide ou des composants amenés à venir en contact avec le liquide, est constituée en totalité ou en partie d'un matériau transparent ou translucide.

8. Dispositif selon l'une quelconque des revendications 4 à 7,
**caractérisé en ce que**
les capteurs sont placés dans une chambre de mesure.

9. Dispositif selon l'une quelconque des revendications 1 à 8,
**caractérisé en ce que**
de la lumière UV est émise aux fins de la détection de dépôts biologiques.

10. Dispositif selon l'une quelconque des revendications 1 à 9,
**caractérisé en ce que**
le système d'analyse détermine l'épaisseur du dépôt biologique et/ou inorganique.
